# EUROPEAN PATENT APPLICATION

(11) **EP 4 603 115 A1**
(43) Date of publication of application: **20.08.2025**
(21) Application number: 24305253.7
(22) Date of filing: 14.02.2024
(51) Int. Cl.: A61M 5/00

(54) **MEDICAL COMPONENT CONTAINER SYSTEM WITH DEGASSING VALVE**

(71) Applicant: Becton Dickinson France, 38800 Le Pont-de-Claix (FR)
(72) Inventor: LE LOC'H, Clémentine, 38240 Meylan (FR); EYMERY, Anaïs, 38450 Saint-Georges-de-Commiers (FR)
(74) Representative: Germain Maureau

(57) **Abstract**

Provided herein is a container system for retaining a plurality of medical components. The container system includes a tub having a bottom wall, sidewalls extending upwardly from the bottom wall, and a top lip formed on the sidewalls, with the top lip defining an upper opening of the tub. The container system also includes one or more sealing elements configured to seal off the interior volume of the tub from an ambient environment, with each of the one or more sealing elements comprising a sealing sheet that is impervious or substantially impervious to a flow of gasses therethrough, along with a degassing valve provided on the sealing sheet, the degassing valve comprising a one-way valve that enables a flow of gasses out from the interior volume of the tub to the ambient environment, but not from the ambient environment into the interior volume of the tub.

## Description

### BACKGROUND OF THE INVENTION

### Field of the Invention

The present disclosure relates generally to containers for the packaging of medical containers or components and, more particularly, to a container system that provides for proof of maintained packaging integrity.

### Description of Related Art

Medical containers and components for use with such containers, such as pre-fillable or prefilled syringes, cartridges, barrels, or stoppers, often need to be transported from one site to another site during or after manufacturing. For instance, medical containers or components thereof may be transported from a manufacturing site to a site at which the medical containers may be filled with a medicinal fluid. As used herein, a "medical fluid" can refer to a medication or another therapeutic agent used for treatment of chronic or acute conditions, as are known in the art. Exemplary therapeutic agents can include, for example, drugs, chemicals, biological, or biochemical substances that, when delivered in a therapeutically effective amount to the patient, achieve a desired therapeutic effect.

Containers or "tubs" are typically used to transport the medical containers or components. In some embodiments, the medical containers or components may be stored loosely in the tub without any additional supporting or holding devices. In other embodiments, the medical containers or components may be supported within the tub by one or more holding devices, such as trays or "nests." As known in the art, nests are available for holding/transporting syringe barrels or stoppers to be inserted in such syringe barrels (such as after pre-filling of a syringe barrel), with the nest(s) disposed within the tub and supported thereby. Upon the tub having received the medical containers or components (and, optionally, the nest(s)), an opening of the tub is sealed. In some embodiments, the tub may be sealed by a sealing cover that is applied directly over a top opening of the tub (e.g., sealed onto an upper flange of the tub body). In other embodiments, the tub may be sealed by a header bag that has an interior volume sized to receive the tub, with the header bag being sealed to enclose the tub therein.

Many tubs used to transport medical containers or components are configured to provide for the sterilization of the medical devices contained therein. That is, after the medical containers or components have been stored and sealed within the tub, a step may be implemented to destroy any contamination within the tub. To that end, the sealing cover of the tub (or at least a portion of the header bag) may be formed of a material that is porous to gases and particularly to sterilization gases (e.g., ethylene oxide) and which thus allows said sterilizing gases to penetrate into the tub to be in contact with the medical devices. As one example, it is common to use a Tyvek^{®} sealing cover (or a header bag with a Tyvek^{®} window therein) to seal the opening of the tub and provide for the subsequent sterilization of medical devices within the sealed tub. After performing of such a sterilization procedure, the sealing cover (or header bag) then functions to preserve the sterility of the medical devices within the tub by providing a barrier that prevents foreign particles or contaminants (e.g., microorganisms, dust, or other particles) from entering inside the tub - with the sealing cover (or header bag) maintaining the integrity of the tub until a subsequent opening of the tub for use of the medical devices.

In addition to providing for the sterilization (and continued integrity) of medical devices within a tub, the porous sealing cover (or porous window in the header bag) also allows for an ongoing circulation of air into and/or out of the tub during shipping. This allowing of air flow circulation into and/or out of the tub can aid in maintaining the sealing of the tub during shipment. That is, it is recognized that pressure within and/or outside of the tub can vary during shipment thereof, such as during air shipment or other high altitude transport of the tub, and by letting air circulate through the sealing cover, there is less risk of the sealing cover on the tub breaking/bursting responsive to a large environmental pressure decrease.

While tubs and their associated porous sealing covers and header bags as described above are effective in providing for the sterilization and shipment of medical devices, it is recognized that porous sealing covers and header bags may be cost prohibitive. As a primary example, Tyvek^{®} sealing covers (or header bags with Tyvek^{®} windows) may be expensive and/or be hard to acquire (due to material/component shortages). Thus, it may be desirable to replace porous sealing covers / header bags with cheaper non-porous sealing covers, but it is recognized that such covers / header bags introduce other challenges - such as those described above regarding maintaining integrity of the sealed tub when experiencing pressure changes during shipment.

Accordingly, it is desirable to provide tubs and associated sealing covers and/or header bags that are cost efficient and yet able to accommodate pressure changes during shipment of the tubs. It is further desirable to provide a means for ensuring sterility of an external surface of the tub and sealing cover.

### SUMMARY OF THE INVENTION

Provided herein is a container system for retaining a plurality of medical components. The container system includes a tub having a bottom wall and a plurality of sidewalls extending upwardly from the bottom wall, with the plurality of sidewalls and the bottom wall defining an interior volume of the tub. The tub also includes a top lip formed on the plurality of sidewalls, with the top lip defining an upper opening of the tub. The container system also includes one or more sealing elements configured to seal off the interior volume of the tub from an ambient environment, with each of the one or more sealing elements comprising a sealing sheet that is impervious or substantially impervious to a flow of gasses therethrough. Each of the one or more sealing elements also includes a degassing valve provided on the sealing sheet, the degassing valve comprising a one-way valve that enables a flow of gasses out from the interior volume of the tub to the ambient environment, but not from the ambient environment into the interior volume of the tub.

In accordance with some aspects of the disclosure, the one or more sealing elements are each composed of polyethylene, polypropylene, and/or polyethylene terephthalate.

In accordance with some aspects of the disclosure, the one or more sealing elements comprises a sealing lid that is secured to the top lip of the tub.

In accordance with some aspects of the disclosure, the one or more sealing elements comprises a header bag configured to receive the tub, with the header bag comprising a sealing end that, when sealed, enclosed the tub therein.

In accordance with some aspects of the disclosure, the one or more sealing elements comprises both the sealing lid and the header bag, with the header bag enclosing the tub with the sealing lid provided thereon, and wherein each of the sealing lid and the header bag includes a respective degassing valve thereon.

In accordance with some aspects of the disclosure, the header bag is in a vacuumed state, with the vacuumed state of the header bag proving an integrity of the header bag and a sterility of the tub.

In accordance with some aspects of the disclosure, the degassing valve accommodates a pressure decrease in the ambient environment responsive to which an air volume within the interior volume of the tub increases, with the degassing valve allowing gas to escape from the interior volume of the tub to the ambient environment.

In accordance with some aspects of the disclosure, the degassing valve comprises an air inlet member comprising one or more openings therein, the air inlet member joined with the sealing sheet, a plastic membrane positioned on the air inlet member and movable between a sealing position and an air outflow position, and an air outlet member positioned within the air inlet member to retain the membrane between the air inlet member and the air outlet member. The membrane separates from the air inlet member to the air outflow position to enable gas to escape from the interior volume of the tub to the ambient environment and remains engaged with the air inlet member at the sealing position to prevent an inflow of gas from the ambient environment into the interior volume of the tub.

In accordance with some aspects of the disclosure, the container system further comprises one or more nests held within the tub and configured to retain a plurality of medical components therein, each of the one or more nests comprising a support plate having a top surface and a bottom surface, and a plurality of chimneys formed on the support plate and configured to retain the plurality of medical components therein, each of the plurality of chimneys comprising a cylindrical wall defining a receptacle within which a respective medical component is retained.

Also provided herein is a method for packaging a plurality of medical components. The method includes placing the plurality of medical components within the interior volume of the tub, sealing the plurality of medical components within the interior volume of the tub and from the ambient environment using the one or more sealing elements, and sterilizing the plurality of medical components and the interior volume of the tub using a sterilization technique.

In accordance with some aspects of the disclosure, sterilizing the plurality of medical components comprises exposing the plurality of medical components to a dose of gamma rays as part of a gamma sterilization.

In accordance with some aspects of the disclosure, sealing the plurality of medical components within the interior volume of the tub comprises securing a sealing lid to the top lip of the tub, with the sealing lid including the degassing valve thereon.

In accordance with some aspects of the disclosure, sealing the plurality of medical components within the interior volume of the tub further comprises applying a header bag over the tub, with the sealing lid secured thereon, the header bag including the degassing valve thereon, and sealing the header bag to enclose the tub therein.

In accordance with some aspects of the disclosure, the method further includes creating a vacuum within the header bag, and wherein confirmation of an integrity of the header bag and a sterility of the tub is ensured when the vacuum is maintained in the header bag.

In accordance with some aspects of the disclosure, the degassing valve accommodates a pressure decrease in the ambient environment responsive to which an air volume within the interior volume of the tub increases, with the degassing valve allowing gas to escape from the interior volume of the tub to the ambient environment.

### BRIEF DESCRIPTION OF THE DRAWINGS

FIG. 1 is a perspective view of a packaging system for storing and shipping a plurality of medical components, according to an embodiment of the disclosure;
FIG. 2 is an exploded view of the packaging system of FIG. 1;
FIGS. 3A and 3B are detailed views of a one-way degassing valve included on a sealing lid of the tub of FIG. 1, showing the valve in an air outflow position and a sealing position, respectively, according to an embodiment of the disclosure;
FIG. 4 is a side view of a packaging system for storing and shipping a plurality of medical components, according to another embodiment of the disclosure; and
FIG. 5 is a side view of a packaging system for storing and shipping a plurality of medical components, according to another embodiment of the disclosure.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

Aspects and embodiments of the disclosure are directed to tubs that are used for transporting a plurality of nests and the medical containers or components held thereby. The tubs are designed so as to provide for the nested stacking of empty tubs during shipping, while minimizing the possibility of tub deformation resulting from such nested stacking.

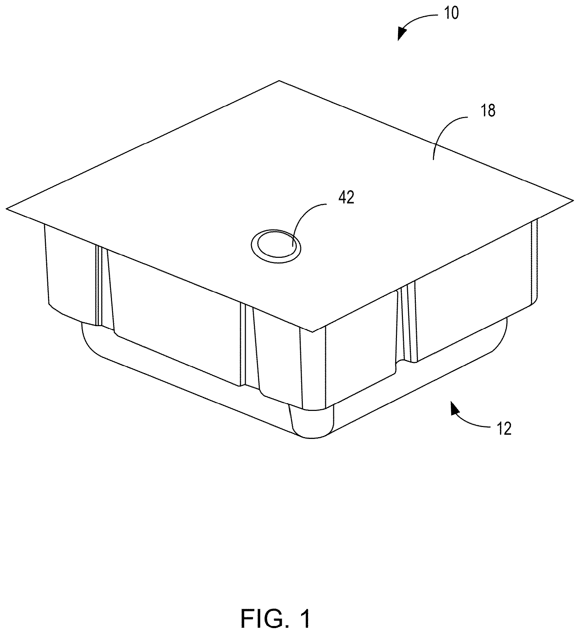

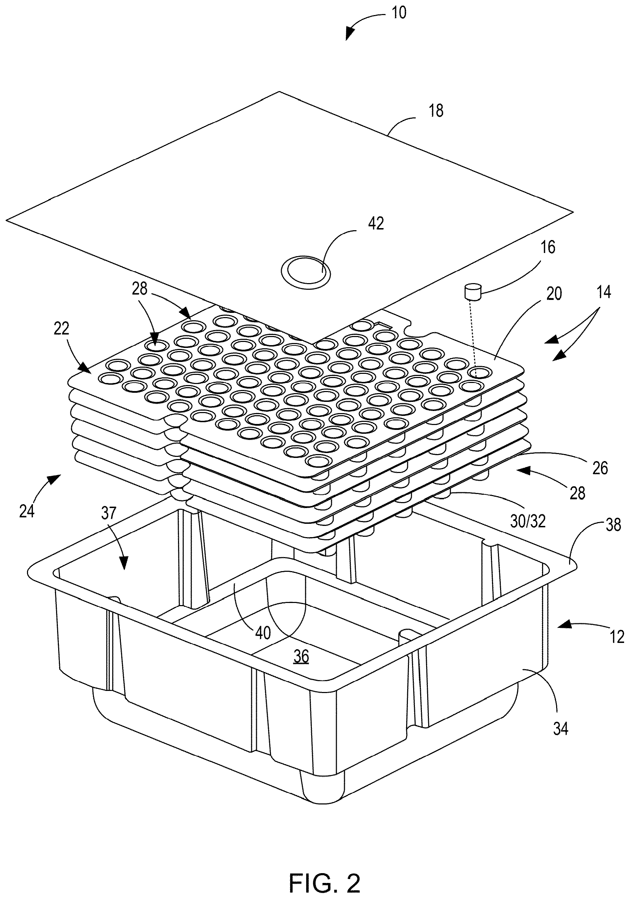

Referring to FIGS. 1 and 2, shown is a non-limiting embodiment of a packaging system 10 that may be used for the packaging and transporting of a plurality of medical components therein. The packaging system 10 includes a container or tub 12 within which a plurality of medical components may be retained. In some embodiments, the packaging system 10 also includes one or more nests 14 that may be housed within the tub 12, with each of the nests 14 retaining a plurality of medical components therein. In the illustrated embodiment, each of nests 14 is configured to retain therein a plurality of stoppers 16 that may be utilized in syringe containers, as known in the art, although it is recognized that nests 14 could alternatively retain other medical containers or components therein - including syringe barrels or assembled pre-filled syringes. The packaging system 10 also includes a sealing element 18 that is configured to seal off an interior volume of the tub 12 from an ambient environment after the nests 14 are packaged therein, to provide a sterile environment for storing and shipping the stoppers 16. In the illustrated embodiment, the sealing element 18 is provided as a sealing lid ("sealing lid 18") that may be applied over the tub 12 after the nests 14 are packaged therein.

Exemplary nests 14 that may be retained in tub 12 are shown in FIG. 2. The nests 14 each comprise a support plate 20 having a first or upper surface 22, a second or lower surface 24, and a peripheral edge 26 extending about the support plate 20 between the upper surface 22 and the lower surface 24. In some examples, the perimeter of support plate 20 is substantially rectangular in shape, such that the nest 14 may fit within the tub 12. The nests 14 further comprise tubular walls, members, or ridges - referred to hereafter as "chimneys 28" - that protrude outward and downward from a plane defined by the upper surface 22 of the support plate 20. Each chimney 28 can include a cylindrical wall 30 that defines a receptacle 32 configured to receive a stopper 16 therein, with the receptacle 32 sized to retain an entirety of the stopper 16. The receptacle 32 includes a top opening at a top end of the chimney 28 and a bottom opening at a bottom end of the chimney 28. As indicated previously, the chimneys 28 may be configured to retain medical components other than stoppers, such as syringe barrels or pre-filled syringes, as non-limiting examples, with it recognized that the chimneys 28 will be specifically configured as appropriate in order to retain such components.

With regard to tub 12, the tub 12 may be formed as a molded plastic tub (e.g., an injection molded polystyrene tub) that includes a plurality of sidewalls 34 that extend upwardly from a bottom wall 36. Each of the sidewalls 34 may be configured as a sloped sidewall that does not extend exactly vertical from bottom wall 36, but instead extends up at an angle therefrom. The sidewalls 34 and the bottom wall 36 collectively define a hollow interior volume 37 of the tub 12. Opposite the bottom wall 36, the tub 12 is open such that the nests 12 may be inserted therein. A top flange 38 may be provided at a top end of the sidewalls 34 opposite to which the bottom wall 36 is located. The top flange 38 is configured to provide for coupling of sealing lid 18 thereto after one or more nests 14 having stoppers 16contained therein are disposed in the tub 12.

In some embodiments, each of the sidewalls 34 may optionally have a stepped construction that defines a ledge 40 positioned between a bottom sidewall portion and a top sidewall portion of each sidewall 34, although it is recognized that other tubs may not have sidewalls configured in this manner. In use of the tub 12, the ledge 40 provides a horizontal surface configured to support the plate 20 of a bottom-most nest 14 such that, when the plate 20 is disposed in the tub 12, the lower surface 24 of the plate 20 is disposed on the ledge 40.

As indicated above, a sealing lid 18 is applied over the tub 12 (i.e., secured to top flange 38) after the nests 14 (and stoppers 16) are packaged therein, to provide a sterile environment for storing and shipping the stoppers 16. According to aspects of the disclosure, the sealing lid 18 of packaging system 10 may be configured to provide for sterilization of the interior of the tub 12 after it has been secured on the tub 12. The sealing lid 18 may also be configured to accommodate pressure changes within the interior volume 37 of the tub 12 during shipment thereof, such as may occur during air shipment of the tub 12 at high altitude, by enabling gas to be vented out from the interior volume 37 of the tub 12. The sealing lid 18 may be provided as a "low cost" lid that is cheaper than conventional tub lids that may be formed of a material that is porous to air and/or gases (e.g., sterilization gases), such as lids formed of Tyvek^{®} and/or including a window of Tyvek^{®}).

According to aspects of the disclosure, the sealing lid 18 is configured as a lid that is impervious or substantially impervious to gas, such as oxygen, air, carbon dioxide and/or water vapor, for instance impervious to air. The sealing lid 18 being impervious or substantially impervious to air is understood to mean that the sealing lid 18 is formed of a material that has little or no permeability to gas such as air or oxygen. For example, by being substantially impervious to air, the permeability of sealing lid 18 to oxygen is a permeability less than 1,000cm3/25µm/24h. Said permeability may be measured by any machine known from the skilled person, for example with a permeation oxygen analyzer (OxyPerm, Ox-Tran 2.22) according to the ASTM D3985 (Standard Test Method for Oxygen Gas Transmission Rate Through Plastic Film and Sheeting Using a Coulometric Sensor) method. To provide such imperviousness to air, the sealing lid 18 may be made of a polymeric material such as polyethylene (in particular low-density linear polyethylene (LLDPE)), polypropylene, and/or polyethylene terephthalate, as non-limiting examples.

With the sealing lid 18 being configured as a lid that is impervious or substantially impervious to gas, sterilization of the interior volume 37 of the tub 12 (and the nests 14 and components 16 retained therein) must be performed by a method other than the application of sterilization gases (e.g., ethylene oxide) through a gas permeable sealing lid. According to aspects of the disclosure, sterilization of the interior volume 37 of the tub 12 may thus instead be performed via use of a gamma sterilization technique - where the interior volume 37 of the tub 12 and the plurality of medical components (e.g., stoppers 16) are exposed to a dose of gamma rays. It is understood that the gamma rays may be directed through the sealing lid 18 and into the interior volume 37 of the tub 12, after the tub 12 has been sealed shut.

According to aspects of the disclosure, the sealing lid 18 includes a degassing valve 42 thereon that allows for pressure within the interior volume 37 of the tub 12 to be controlled. The degassing valve 42 is configured as a one-way air valve that enables a flow of air out from the interior volume 37 of the tub 12 to the ambient environment, but does not allow a flow of air from the ambient environment into the interior volume 37 of the tub 12. Via inclusion of the degassing valve 42, the sealed tub 12 is able to accommodate a pressure decrease in the ambient environment (such as may occur at high altitude during air shipment), responsive to which an air volume within the interior volume 37 of the tub 12 increases - with the degassing valve 42 allowing air to escape from the interior volume 37 of the tub 12 to the ambient environment. This releasing of air from the interior volume 37 of the tub 12 can prevent the sealing lid 18 from bursting were the air volume within the interior volume 37 of the tub 12 to increase too much.

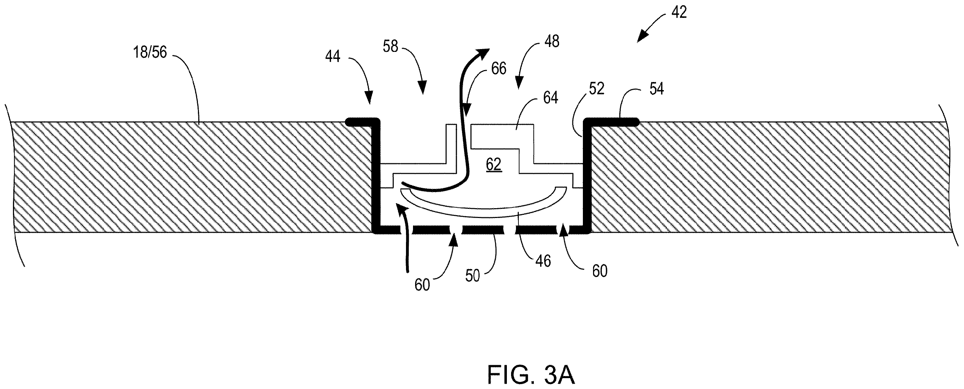

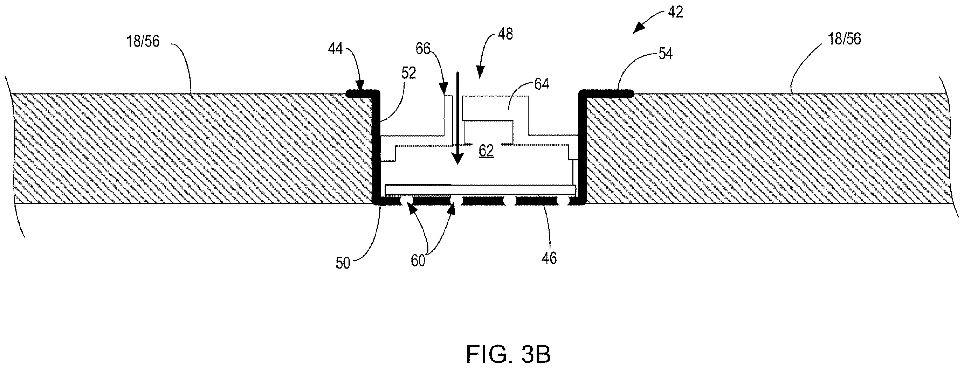

FIGS. 3A and 3B illustrate the construction of the degassing valve 42 in more detail, according to embodiment of the disclosure. The degassing valve 42 has a 3-piece construction - with the valve 42 including an air inlet member 44, a membrane 46, and an air outlet member 48. Each of the inlet member, membrane 46, and air outlet member 48 may be formed of a polymeric material (i.e., plastic) that is impervious to air flow therethrough.

The air inlet member 44 is configured as a generally cup-shaped member with a bottom wall 50 and a cylindrical sidewall 52. In some embodiments, an annular flange 54 is formed at the top of the sidewall 52 that provides for securing of the air inlet member 44 (and degassing valve 42) to a sealing sheet 56 that forms sealing lid 18. The air inlet member 44 may be secured within an opening 58 formed in sealing sheet 56, with the cylindrical sidewall 52 extending through the opening 58 and the annular flange 54 being secured (e.g., glued) to an outer surface of the sealing sheet 56 (i.e., surface opposite from the interior volume 37 of tub 12). The bottom wall 50 of air inlet member 44 includes a plurality of openings or holes 60 formed therein through which air may flow, to enable air to be vented out from the interior volume 37 of tub 12.

The air outlet member 48 is sized to fit within an interior chamber/volume 62 of air inlet member 44. The air outlet member 48 may be configured as a circular or disc-shaped member having an outer diameter that is essentially equal to an inner diameter of the interior chamber/volume 62 of air inlet member 44 - such that a seal is provided between the circumference of the air outlet member 48 and the inner surface of the cylindrical sidewall 52 of air inlet member 44. The air outlet member 48 may have a raised central portion 64 that includes an opening or slot 66 therein through which air may flow, to enable air to be vented out from the interior volume 37 of tub 12.

The membrane 46 is positioned within the interior chamber/volume 62 of air inlet member 44, with the air outlet member 48 positioned over the membrane 46 such that the membrane 46 is located between the air outlet member 48 and the bottom wall 50 of air inlet member 44. The membrane 46 is movable within a spaced defined between the air outlet member 48 and the bottom wall 50 of air inlet member 44 - with the membrane 46 being movable between what are referred to as a "sealing position" and an "air outflow position." With the membrane 46 in the air outflow position, as shown in FIG. 3A, the membrane 46 moves away from the bottom wall 50 of the air inlet member 44 so as to be separated therefrom - such that the openings/holes 60 formed in the bottom wall 50 are no longer covered and air is allowed to flow through the openings/holes 60.

In use of the tub 12, in situations where a volume of the air within the interior volume 37 of tub 12 has expanded, air within the interior volume 37 of tub 12 may be under pressure and cause the membrane 46 to move from the sealing position to the air outflow position. As the membrane 46 separates/lifts from the bottom wall 50 of air inlet member 44 and moves to the air outflow position (FIG. 3A), air from within the interior volume 37 of tub 12 flows out through the openings/holes 60 in the bottom wall 50, with the air then flowing around the membrane 46 and out through the slot 66 formed in air outlet member 48. The air is thus vented to the ambient environment, thereby lowering the air pressure within the interior volume 37 of tub 12. With the air pressure within the interior volume 37 of tub 12 thus lowered, the membrane 46 is able to move back to the sealing position (FIG. 3B), where the membrane 46 is in contact with the bottom wall 50 of air inlet member 44 and covers the openings/holes 60 therein. Accordingly, the membrane 46 prevents air from flowing from the ambient environment into the interior volume 37 of tub 12.

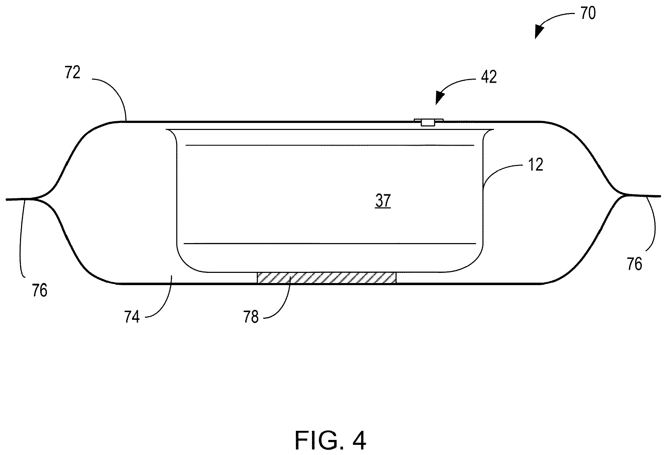

Referring now to FIG. 4, according to another embodiment of the disclosure, rather than including a sealing lid 18 that is secured directly to the tub 12, the container system 10 may instead include a sealing element in the form of a header bag 70 that is to be positioned about the tub 12 and form an enclosure thereabout. The header bag 70 comprises at least one sealing sheet that is configured to be sealed to form an enclosure about the tub 12. In the illustrated embodiment, the header bag 70 comprises two sheets 72, 74 that are sealed together, but it is recognized that the header bag 70 could instead be formed of a single sheet that is folded on itself and then sealed along its periphery 76. The sealing of the sheets 72, 74 may be carried out by locally heating a region of the periphery 76 of the sheets 72, 74 and applying a mechanical pressure to promote welding of the materials of the two sheets 72, 74 along a continuous line. In some embodiments, the header bag 70 (e.g., sheet 74) may be adhered to the bottom of the tub 12 by a double-sided tape 78, so as to secure the tub 12 relative to the header bag 70.

According to embodiments of the disclosure, each of the sheets 72, 74 is configured to be impervious or substantially impervious to gas, such as oxygen, air, carbon dioxide and/or water vapor, for instance impervious to air. As previously described, the sealing sheets 72, 74 being impervious or substantially impervious to gas is understood to mean that the sealing sheets 72, 74 are formed of a material that has little or no permeability to gas such as air or oxygen. For example, by being substantially impervious to air, the permeability of the sealing sheets to oxygen is a permeability less than 1,000cm3/25µm/24h. To provide such imperviousness to air, the sealing sheets 72, 74 may be made of a polymeric material such as polyethylene (in particular low-density linear polyethylene (LLDPE)), polypropylene, and/or polyethylene terephthalate, as non-limiting examples.

With the sealing sheets 72, 74 being configured as sheets that are impervious or substantially impervious to gas, sterilization of the interior volume 37 of the tub 12 (and the nests 14 and components 16 retained therein) must be performed by a method other than the application of sterilization gases (e.g., ethylene oxide) through a gas permeable header bag 70. According to aspects of the disclosure, sterilization of the interior volume 37 of the tub 12 may thus instead be performed via use of a gamma sterilization technique - where the plurality of medical components are exposed to a dose of gamma rays. It is understood that the gamma rays may be directed through the sealing sheets 72, 74 and into the interior volume 37 of the tub 12, after the tub 12 has been enclosed within the header bag 70.

According to aspects of the disclosure, at least one of the sealing sheets 72, 74 (e.g., sealing sheet 72) includes a degassing valve 42 thereon that allows for pressure within the interior volume 37 of the tub 12 to be controlled. The degassing valve 42 is configured as previously described - with the valve 42 being a one-way air valve that enables a flow of air out from the space enclosed by the header bag 70, but does not allow a flow of air from the ambient environment into the header bag 70. Via inclusion of the degassing valve 42, the enclosed tub 12 is able to accommodate a pressure decrease in the ambient environment (such as may occur at high altitude during shipment), responsive to which an air volume within the header bag 70 increases - with the degassing valve 42 allowing air to escape from the header bag 70 to the ambient environment and preventing a potential bursting of the header bag 70.

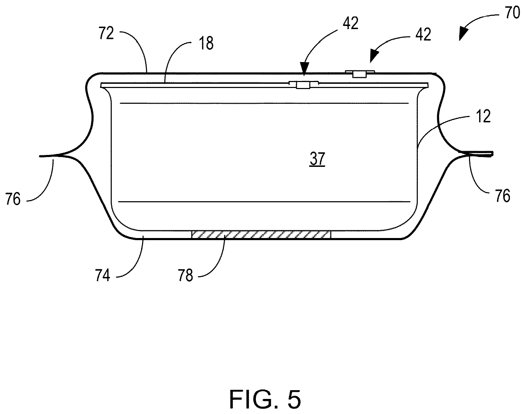

In accordance with some aspects of the disclosure, the container system 10 may include a tub 12 having a sealing lid 18 secured thereto, as a well as a header bag 70 that encloses the sealed tub 12, as shown in FIG. 5. Each of the sealing lid 18 and the header bag 70 may be constructed as previously described, with the sealing lid 18 and the header bag 70 each formed of sheets/materials configured to be impervious or substantially impervious to air, and with sealing lid 18 and the header bag 70 each including a degassing valve 42 thereon. With the arrangement of the sealed tub 12 being enclosed within the header bag 70, the header bag 70 can function as an indicator regarding packaging integrity and sterility of the tub 12, as described in further detail below.

According to aspects of the disclosure, a vacuum within the header bag 70 can be created during or after sealing together of the sheets 72, 74 of the header bag 70, such that the header bag 70 can function as an indicator regarding packaging integrity and sterility of the tub 12. For example, the vacuum may be created thanks to vacuum sealing machine such as MAGVAC^{™} machines. According to aspects of the disclosure, creating of a vacuum within the header bag 70 is defined as providing a pressure less than 340 mbar, or preferably less than 250 mbar, for instance a pressure comprised between 50 mbar and 250 mbar. When such a vacuum is created, the sheets 72, 74 of header bag 70 will conform (at least somewhat) to the shape of the sealed tub 12, with the conforming of the header bag 70 to the shape of the tub 12 indicating that the integrity of the header bag 70 is intact (i.e., it remains in a vacuumed state).

With the header bag 70 vacuumed about the sealed tub 12, it is recognized that any air that is vented out from the interior volume 37 of the tub 12 - through the degassing valve 42 on sealing lid 18 - would be vented out into the volume enclosed by the header bag 70. This air that is vented into the header bag 70 could cause the header bag 70 to look inflated (indicating, potentially, that the integrity of the header bag 70 has been compromised); however, the degassing valve 42 on the header bag 70 allows for any air within the header bag 70 to be vented out therefrom to the ambient environment. The degassing valve 42 on the header bag 70 thus enables the header bag 70 to remain in its vacuumed state, thus indicating that the integrity of the header bag 70 is intact and that the sterility of the tub 12 (and the nests 14 and medical components 16 therein) has been maintained.

Beneficially, embodiments of the invention thus provide container systems that are used for transporting a plurality of nests and the medical containers or components held thereby. The container system may include a tub within which the nests and the medical containers are retained, along with one or more sealing elements configured to seal off the interior volume of the tub from an ambient environment. In some embodiments, a sealing lid is applied over the tub, with the sealing lid comprising a sealing sheet that is impervious or substantially impervious to a flow of air therethrough, and with the sealing lid including a one-way degassing valve that allows air to be vented out from the interior volume of the tub. In some embodiments, a header bag is provided that encloses the tub, with the header bag comprising sealing sheet(s) that are impervious or substantially impervious to a flow of air therethrough, and with the a sealing sheet including a one-way degassing valve that allows air to be vented out from the interior volume of the tub. In some embodiments, the container system may include both a sealing lid on the tub and a header bag about the tub, as previously described, with the header bag being in a vacuumed state and generally conforming to the tub. With the header bag in the vacuumed state, and with degassing valves provided on both the sealing lid and the header bag, the presence of a continued vacuum within the header bag serves as an indicator regarding packaging integrity and sterility of the tub.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A container system for retaining a plurality of medical components, the container system comprising:
a tub including:
a bottom wall; and
a plurality of sidewalls extending upwardly from the bottom wall, with the plurality of sidewalls and the bottom wall defining an interior volume of the tub; and
a top lip formed on the plurality of sidewalls, with the top lip defining an upper opening of the tub; and
one or more sealing elements configured to seal off the interior volume of the tub from an ambient environment, with each of the one or more sealing elements comprising a sealing sheet that is impervious or substantially impervious to a flow of gasses therethrough;
wherein each of the one or more sealing elements comprises a degassing valve provided on the sealing sheet, the degassing valve comprising a one-way valve that enables a flow of gasses out from the interior volume of the tub to the ambient environment, but not from the ambient environment into the interior volume of the tub.

2. The container system of claim 1, wherein the one or more sealing elements are each composed of polyethylene, polypropylene, and/or polyethylene terephthalate.

3. The container system of claim 1 or claim 2, wherein the one or more sealing elements comprises a sealing lid that is secured to the top lip of the tub.

4. The container system of any of claims 1-3, wherein the one or more sealing elements comprises a header bag configured to receive the tub, with the header bag comprising a sealing end that, when sealed, enclosed the tub therein.

5. The container system of claim 3 or claim 4, wherein the one or more sealing elements comprises both the sealing lid and the header bag, with the header bag enclosing the tub with the sealing lid provided thereon, and wherein each of the sealing lid and the header bag includes a respective degassing valve thereon.

6. The container system of claim 5, wherein the header bag is in a vacuumed state, with the vacuumed state of the header bag proving an integrity of the header bag and a sterility of the tub.

7. The container system of any of claims 1-6, wherein the degassing valve accommodates a pressure decrease in the ambient environment responsive to which an air volume within the interior volume of the tub increases, with the degassing valve allowing gas to escape from the interior volume of the tub to the ambient environment.

8. The container system of any of claims 1-7, wherein the degassing valve comprises:
an air inlet member comprising one or more openings therein, the air inlet member joined with the sealing sheet;
a plastic membrane positioned on the air inlet member and movable between a sealing position and an air outflow position; and
an air outlet member positioned within the air inlet member to retain the membrane between the air inlet member and the air outlet member;
wherein the membrane separates from the air inlet member to the air outflow position to enable gas to escape from the interior volume of the tub to the ambient environment; and
wherein the membrane remains engaged with the air inlet member at the sealing position to prevent an inflow of gas from the ambient environment into the interior volume of the tub.

9. The container system of any of claims 1-8, wherein the container system further comprises one or more nests held within the tub and configured to retain a plurality of medical components therein, each of the one or more nests comprising:
a support plate having a top surface and a bottom surface; and
a plurality of chimneys formed on the support plate and configured to retain the plurality of medical components therein, each of the plurality of chimneys comprising a cylindrical wall defining a receptacle within which a respective medical component is retained.

10. A method for packaging a plurality of medical components using the container system of claim 1, the method comprising:
placing the plurality of medical components within the interior volume of the tub;
sealing the plurality of medical components within the interior volume of the tub and from the ambient environment using the one or more sealing elements; and
sterilizing the plurality of medical components and the interior volume of the tub using a sterilization technique.

11. The method of claim 10, wherein sterilizing the plurality of medical components comprises exposing the plurality of medical components to a dose of gamma rays as part of a gamma sterilization.

12. The method of claim 10 or claim 11, wherein sealing the plurality of medical components within the interior volume of the tub comprises securing a sealing lid to the top lip of the tub, with the sealing lid including the degassing valve thereon.

13. The method of claim 12, wherein sealing the plurality of medical components within the interior volume of the tub further comprises:
applying a header bag over the tub, with the sealing lid secured thereon, the header bag including the degassing valve thereon; and
sealing the header bag to enclose the tub therein.

14. The method of claim 13, further comprising creating a vacuum within the header bag, and wherein confirmation of an integrity of the header bag and a sterility of the tub is ensured when the vacuum is maintained in the header bag.

15. The method of any of claims 10-14, wherein the degassing valve accommodates a pressure decrease in the ambient environment responsive to which an air volume within the interior volume of the tub increases, with the degassing valve allowing gas to escape from the interior volume of the tub to the ambient environment.
